(19) Europäisches Patentamt
European Patent Office
Office européen des brevets

(11) Publication number: 0 334 338 A2

(12) # EUROPEAN PATENT APPLICATION

(21) Application number: 89105173.2

(22) Date of filing: 22.03.89

(51) Int. Cl.4: A61K 6/08 , C08F 4/00

(30) Priority: 24.03.88 US 173388

(43) Date of publication of application: 27.09.89 Bulletin 89/39

(84) Designated Contracting States: AT BE CH DE ES FR GB GR IT LI LU NL SE

(71) Applicant: DENTSPLY INTERNATIONAL, INC.
570 West College Avenue P.O. Box 872
York Pennsylvania 17405(US)

(72) Inventor: Whitten, David
72 Canterbury Road
Rochester, NY 14607(US)
Inventor: Venz, Sabine
19223 Dunbridge Way
Gaitherburg, MD 20897(US)

(74) Representative: Wächtershäuser, Günter, Dr.
Tal 29
D-8000 München 2(DE)

(54) **Titanate initiators for light cured compositions.**

(57) Titanate initiators for light cured and dual cure compositions are provided. In the preferred embodiment, the initiators of the invention will be used in a composition comprising an unsaturated monomer, and the preferred initiator is Bis-pentafluorophenyl Bis-cyclo- pentadienyl titanate. A method of using the composition for treating teeth by applying it to a prepared tooth is also provided.

EP 0 334 338 A2

# TITANATE INITIATORS FOR LIGHT CURED COMPOSITIONS

## BACKGROUND OF THE INVENTION

This application is a continuation-in-part of U.S. Serial No. 173,388, filed March 24, 1988

This invention relates to initiators used with dental compositions which are curable by free radical polymerization and, preferably, by the action of visible light. More particularly, use of the initiators with one-component dental composite formulations and urethane impression material formulations are disclosed.

Dental restoratives should exhibit certain obligatory physical and chemical characteristics in order to be suitable for use in filling, repairing or replacing teeth. Thus, restorative materials should possess properties that closely match natural teeth with respect to structural properties such as cohesive strength, coefficient of thermal expansion and wearability. Also, aesthetic considerations such as color stability, refractive index, plaque repellency, polishability and opacity are important factors in determining whether a mateial is suitable for use as a dental restorative. In the past, numerous compositions have been tried in various mixtures and proportions in order to find satisfactory materials for use as dental composites or restoratives. These compositions have usually included some type of resin, which may either be preblended or mixed by the practitioner in the office, together with other materials such as pigments, catalysts, handling agents and opacifiers. For restorative use, materials generally are "filled", that is, they contain inorganic, or in some cases, organic particulate material. Unfilled, these same resins are used as coatings, especially pit and fissure sealants in preventative dentistry procedures.

Examples of such polymerizable resins include those described in copending U.S. application 040, 636 filed April 21, 1987 and used as described in U.S. Patent 4,514,174 and similar resins as known in the art cited and described in those documents. These resins may be used for producing rigid or elastomeric materials having a wide variety of use in dentistry.

Examples are urethane diacrylates which are used as dental impression materials, dental filling materials, pit and fissure sealers, bridge materials, denture construction materials, drug delivery systems and intraoral bandages. Essentially such compositions include a free radical polymerizable resin and contain polymerization initiators, accelerators and optionally fillers and stabilizers.

It will be appreciated by those skilled in the art that the use of one-component photoactivated materials, ready to use without mixing, are to be preferred over more traditional thermochemical catalyst or redox activated compositions because of the increased work time allowed by the use of photoinitiated polymerization. In a two-component catalyst or redox system, work time is determined by the reaction time once the catalyst is added to the resin component. In a one-component photocured system, the practitioner may take whatever time is necessary for forming or molding a dental impression or restoration into the tooth formation, and then effect extremely rapid curing by exposing the photocurable material to the appropriate wavelength of electromagnetic radiation. Moreover, with a one-component photocured system, the practitioner need not be concerned with properly measuring and mixing two-components to insure that the resultant cured compositions achieve the proper physical characteristics.

There are prior art dental materials which utilize photoinitiators that are sensitive to visible light having wavelengths from about 3,600 angstroms to about 6,000 angstroms. These materials generally would be preferred over those materials which are cured with ultraviolet radiation because visible light is attenuated to a lesser degree by tooth structure than is ultraviolet radiation. However, many previous attempts to develop dental formulations using visible light curing formulations have resulted in less than most desirable performance.

Accordingly, it is a principal object of this invention to provide light cured compositions which are useful for dental applications.

A further object is to provide dental compositions which provide products of high strength, exhibit a rapid cure time, but which exhibit good workability prior to curing.

Still other objects and advantages of the present invention will become apparent from the following description of the invention.

## SUMMARY OF THE INVENTION

In accordance with the invention a photopolymerizable dental composition comprising an olefinically

unsaturated monomer and a photoinitiator comprising cyclopentadienyl titanocenes containing bis-pentafluorobenzene substituents in which one or two carboxylic or heterocyclic aromatic rings are attached to the metal atom, are provided. The composition preferably includes at least one acrylic or methacrylic ester and may include a tertiary amine and/or an oxidizing agent such as benzoylperoxide, cumene hydroperoxide t-butylperbenzoate, acyl phenyl phosphonate or the like.

Proton donating compounds may optionally be added to the composition to enhance the percentage of conversion of the polymer materials.

Also provided is a method of use of the above compositions for treating teeth by its application to a prepared tooth.

It has been found that the initiators of the invention provide a quicker, deeper cure than initiators used in the prior art.

## DETAILED DESCRIPTION OF THE INVENTION

In general, the properties of a resin used for dental applications, especially as a component of a composite, are improved when there is high conversion of monomer (i.e. the monomer substantially is completely cross-linked into the resulting polymer).

In some prior art light cured compositions, although a very good conversion of monomer can be obtained at the surface, the percentage of conversion of monomer decreases at greater depths in the resin or composite materials. (The percentage of conversion is measured spectrographically by quantifying the strength of spectrographic peaks corresponding to carbon-carbon unsaturation in the resin.) It has been theorized that the percentage of conversion decreases at greater depths in the resin because of a combination of the dispersion of light at greater depths, refraction, and the presence of oxygen which is a free radical scavenger which captures free radicals and short circuits or inhibits the free radical chain reaction initiated by the light source.

It is believed that the titanate initiators of the present invention achieve a better depth of cure and higher percentage of conversion at greater depths in the polymer.

The present invention therefore relates to titanates of the formula I

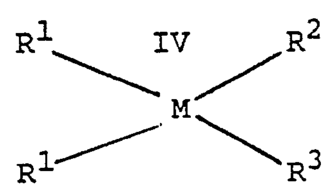

wherein

M is the tetravalent titanium atom,

$R^1$ is each independently unsubstituted or substituted cyclopentadienyl, indenyl or both symbols $R^1$ together denote an unsubstituted or substituted radical of the formula II

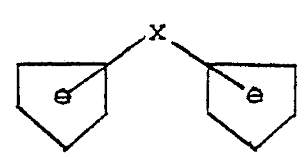

wherein X is $(CH_2)_n$ in which n is 1, 2 or 3, $C_2$-$C_{12}$alkylidene, cycloalkylidene containing 5 to 7 ring carbon atoms, $SiR^4{}_2$ or $SnR^4{}_2$, in which $R^4$ is $C_1$-$C_{12}$alkyl, $C_5$-$C_{12}$cycloalkyl, $C_6$-$C_{16}$aryl or $C_7$-$C_{16}$aralkyl,

$R^2$ is a 6-membered carbocyclic or 5- or 6-membered aromatic or heterocyclic aromatic ring which is substituted by fluorine atoms in at least one of the two ortho-positions relative to the metal carbon bond and which may contain further substituents, or $R^2$ and $R^3$ together are a radical of the formula III

-Q-Y-Q

wherein Q is a carbocyclic or heterocyclic 5- or 6-membered aromatic ring, and each of the two bonds is in the ortho-position to the Y group and each meta-position to the Y group is substituted by a fluorine atom,

and Q may contain further substituents, and Y is $CH_2$, $C_2$-$C_{12}$alkylidene, cycloalkylidene containing 5 to 7 ring carbon atoms, a direct bond, $NR^4$, O, S, SO, $SO_2$, CO, $SiR_2{}^4$ or $SnR_2{}^4$,

$R^3$ is alkynyl, substituted or unsubstituted phenylalkynyl, $N_3$, CN, $SiR_3{}^4$ or has the meaning of $R^2$.

The $R^1$ groups are preferably identical. Suitable substituents for $R^1$ are: linear or branched alkyl, alkoxy and alkenyl of preferably 1 to 18, especially 1 to 12 and most preferably 1 to 6, carbon atoms, e.g. methyl, ethyl, propyl, isopropyl, n-butyl, tert-butyl, pentyl, hexyl, octyl, decyl, dodecyl, tetradecyl, hexadecyl, octadecyl and corresponding alkenyl and alkoxy groups; cycloalkyl and cycloalkenyl containing preferably 5 to 8 ring carbon atoms, e.g. cyclopentyl, cyclohexyl, cycloheptyl, methylpentyl and methylcyclohexyl; aryl of preferably 6 to 16 carbon atoms and aralkyl of preferably 7 to 16 carbon atoms, e.g. phenyl, naphthyl, pyridinyl, benzyl and phenylethyl; nitrilo halogen, preferably F, Cl and Br, and also amino, preferably tertiary amino which may contain linear or branched alkyl groups of 1 to 12, preferably 1 to 6, carbon atoms, in particular methyl or ethyl, or phenyl and benzyl, which amino groups can also be quaternised, in particular with linear or branched alkyl halides containing preferably 1 to 12 carbon atoms, preferably methyl or ethyl halides; linear or branched aminoalkyl, preferably tertiary aminoalkyl which may also be quaternised, in particular with alkyl halides, and the alkylene group in the aminoalkyl can be linear or branched and contains preferably 1 to 12, most preferably 1 to 6, carbon atoms and is most preferably methylene which can be substituted by $C_1$-$C_{12}$alkyl.

The radicals $R^1$ may contain up to 3 substituents, but preferrably contain one substituent. It is preferred than both substituents $R^1$ are cyclopentadienyl or methylcyclopentadienyl.

X in formula II as alkylidene preferably contains 2 to 6 carbon atoms. Exemplary of alkylidene and cycloalkylidene are ethylidene, propylidene, butylidene, hexylidene, phenylmethylene, diphenlmethylene, cyclopentylidene and cyclohexylidene. $R^4$ as alkyl in the group X preferably contains 1 to 6 carbon atoms, e.g. methyl, ethyl, propyl, butyl, or hexyl; and as cycloalkyl is preferably cyclopentyl or cyclohexyl; and as aryl is preferably phenyl; and as aralkyl is preferably benzyl. Most preferably X is methylene.

$R^2$ as 6-membered carbocyclic aromatic and fluorine-substituted ring may be indene, indane, fluorene, naphthalene and preferably phenyl. Preferably both ortho-positions are substituted by fluorine. Exampes are: 4,6-difluoroinden-5-yl, 5,7-difluorind6-yl, 2,4-difluorofluoren-3-yl, 1,3-difluoronaphth-2-yl, and preferably, 2,6-difluorophen-1-yl.

$R^2$ as heterocyclic aromatic 5-membered ring preferably contains one hetero-atom and, as 6-membered ring, preferàbly 1 or 2 hetero-atoms. Examples of such rings substituted by two fluorine atoms are: 2,4-difluoropyrr-3-yl, 2,4-difluorofur-3-yl, 2,4-difluorothiophen-3-yl, 2,4-difluoropyrid-3-yl, 3,5-difluoropyrid-4-yl and 4,6-difluoropyrimid-5-yl.

$R^2$ and $R^3$ together as radical of the formula III may be e.g.:

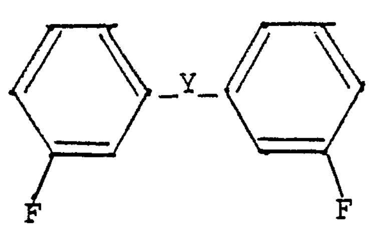 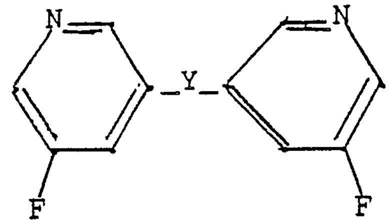

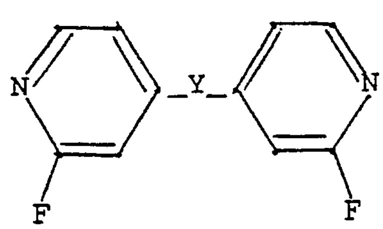 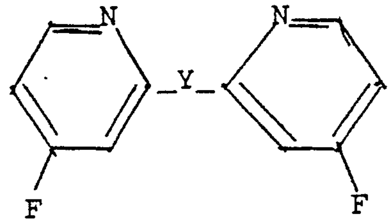

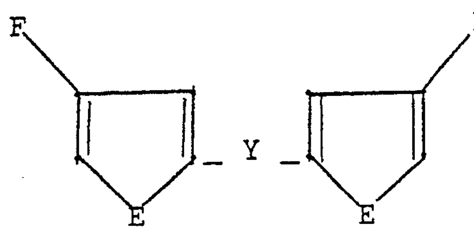 and

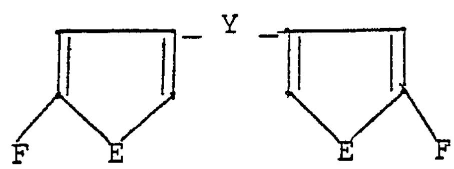

wherein E is O, S or NH, Y in formula III and in the above formulae is preferably methylene, ethylidene, propylidene, a direct bond, S or O.

The radicals $R^2$ can be partly or completely substituted by further groups. Suitable groups are: linear or branched alkyl or alkoxy, each of 1 to 18, preferably 1 to 6 carbon atoms, e.g. methyl, ethyl, propyl, butyl, pentyl, hexyl, and the corresponding alkoxy groups, with methyl and methoxy being preferred; cycloalkyl containing preferably 5 or 6 ring carbon atoms, aryl of preferably 6 to 16 carbon atoms and aralkyl of preferably 7 to 16 carbon atoms, e.g. cyclopentyl, cyclohexyl, phenyl or benzyl; hydroxyl, carboxyl, CN, halogen such as F, Cl or Br, and amino, preferably tertiary amino which may be quaternized with an alkyl halide such as methyl chloride, methyl bromide or methyl iodide, examples of amino groups being methylamino, ethylamino, dimethylamino, diethylamino, pyrrolidyl, piperidyl, piperazyl, morpholinyl, N-methylpiperazyl; alkoxycarbonyl containing preferably 1 to 18, most preferably 1 to 6, carbon atoms in the alkoxy moiety, aminocarbonyl containing one or two $C_1$-$C_{12}$-alkyl groups in the amino group, or aminocarbonyl containing heterocyclic amines such as pyrrolidine, piperdine, piperazine, N-methylpiperazine, and morpholine; aminoalkyl, especially aminoalkyl which preferably contains $C_1$-$C_6$alkyl groups and which may be quaternised with an alkyl halide, most preferably tertiary aminoalkyl which may be substituted by $C_1$-$C_{12}$ alkyl, e.g. dimethylaminomethyl and trimethylammoniummethyl iodide.

Examples of substituents for $R_3$ as phenylalkynyl are halogen such as F, Cl, Br, tertiary amino, $C_1$-$C_6$alkyl and $C_1$-$C_6$alkoxy, carboxyl, OH and CN. $R^3$ preferably has the meaning of $R^2$. Such compounds are described in U.S. patent 4,590,287 which is incorporated herein by reference.

It has been found that Bis-pentafluorophenyl Bis-cyclopentadienyl titanate having the structure

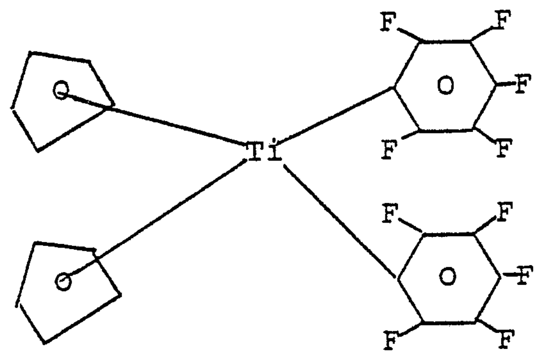

is a very reactive initiator for photocurable dental compositions that is more reactive than camphorquinone for the same purpose. Bis-pentafluorophenyl Bis-cyclopentadienyl titanate has a bleaching effect that allows for greater depth of cure, i.e. Bis-pentafluorophenyl Bis-cyclopentadienyl titanate (PFT) has a yellow/orange color that is bleached when photoirradiated at 360-600 nm, which improves the depth of the cure by its becoming transparent.

Bis-pentafluorophenyl Bis-cyclopentadienyl titanate (PFT) is available from Ciba-Geigy known as MR-97. It has been found that Bis-pentafluorophenyl Bis-cyclo pentadienyl titanate will act as a photoinitiator at about 360-600 nm or without an amine accelerator.

The activity of PFT to visible light irradiation for photopolymerization of resin systems commonly used in dentistry is greater than that of camphorquinone (CQ). Various levels of PFT were tried in the composite and it was determined that .15% by weight PFT gave a cure depth of about 7 mm but rendered the resin systems very yellow in color. Typically, 0.0375% CQ by weight is used to cure compositions and thus reducing the concentration of PFT to only 0.05% by weight (which is about 2/5th the molar amount of CQ used) provides a slightly yellow, stable compositions that, when irradiated with a PRISMETICS® LITE apparatus, gives much better depth of cure than a comparable system using CQ alone (i.e., no amine reducing agent is used in either system). Thus, 0.05% PFT by weight provides a cure depth of 3-5 mm while 0.0375% by weight CQ provides a cure depth of only 1-2 mm. Concentrations as low as 0.0125% by weight PFT have been shown to be effective in combination with other light curing coinitiators, such as peroxide and phosphonate initiators such as those described in copending Attorney Docket Case 1590, incorporated herein by reference.

It has been found that by combining peroxides with PFT, one obtains a post-curing system. That is, initial exposure to visible light provides an initial depth of cure which continues after the light source is removed. For example, a composite system comprising 75% by weight milled barium inorganic glass, 25% by weight resin containing 0.15% by weight PFT and 0.25% by weight cumene hydroperoxide provides an initial depth of cure of 4.9 mm after 10 seconds of irradiation using a PRISMETICS® LITE apparatus. When the specimen was tested 24 hours later, the cure was then 12 mm. Other peroxides are thought to be equally effective, such as benzoyl peroxide (either added directly or coated on inorganic glass filler), t-butyl perbenzoate, per maleic acid (per esters) etc. As another example, a system comprising 0.2% by weight PFT, 0.8% by weight bezoin methyl ether and barium glass, coated with 1% benzoylperoxide (BPO), gave an initial cure of 7.7 mm and a post cure of 11.2 mm (maximum depth of mold used to measure depth of cure).

Further, the acylphenyl phosphonates (i.e., Lucerin-type material of copending case 1590) are also compatible with these systems and provide further enhancements. Thus, for example, a composite system comprising 75% barium boro aluminum silica glass coated with 1% BPO, 23.4% resin, 0.6% PFT and 1% Lucerin had an initial depth of cure of 5.0 mm in 10 seconds using a PRISMETICS® LITE apparatus, and a post cure (measured 24 hours later) depth of 9.0 mm, indicating that once initiated, cure continued after removal of the initiating light.

Amine reducing agents are suitable for enhancing the depth of cure and post cure. Amines are particularly useful when used with diketo initiators in the composition.

It has been found that the addition of proton donating compounds to the composite compositions increases the depth of cure as compared to similar compositions which use only PFT as an initiator and compositions which use PFT in combination with LR as an initiator system. Among the preferred proton donating compound which can be used in the composition are acids and more preferred are organic acids. Illustrative organic acids which may be used in the composition are 3,5-bis-(trifluoromethyl) benzoic acid (BFA), and p-toluenesulfonic acid (PTSA).

Other suitable proton donating compounds will be apparent to those skilled in the art.

The effect of PFT has been characterized using an NCO/TEGDMA resin blend. NCO/TEGDMA is a resin blend described in copending SN 040, 636 and used as illustrated in U.S. Patent 4,514,174 (both incorporated herein by reference) and comprises a blend of NCO, the reaction product of hexamethylene diisocyanate (HMDI) and Bis-GMA

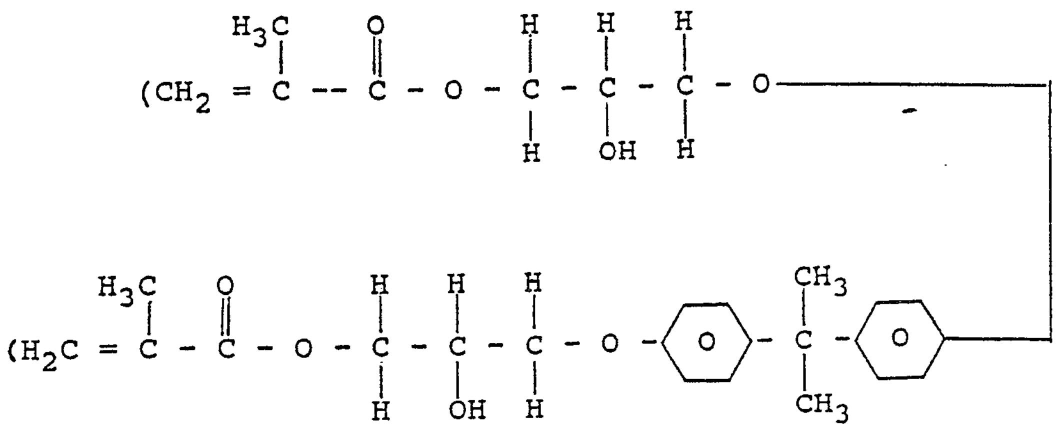

, and triethyleneglycol dimethacrylate (TEGDM). It has been found that PFT works well as an initiator alone, but when used in combination with camphorquinone and an amine, the curing depth and degree of conversion is synergistically improved over a comparable system using only camphorquinone as an initiator and an amine as an accelerator.

As used herein, an amine used in the composition may be any amine known in the art for use as an accelerator in photocurable compositions. Preferably such an amine will have from 1-3 substituents, and will preferably be a tertiary amine, where in the substituents are selected from lower alkyl, acyl, aryl, ester or lower alkyl ester groups.

As used herein, lower alkyl may be a saturated or unsaturated carbon chain having 1-12 carbon atoms, aryl may have 6-12 carbon atoms and acyl and ester may have 1-12 carbon atoms and may include aryl substituents.

As used herein, "heterocyclic" group denotes a 5 to 7 membered aromatic or non-aromatic ring having carbon and 1-4 heteroatoms selected from N, O, S, Si, Se and mixtures thereof. More preferred are rings containing carbon N, O, S or mixtures thereof.

The higher degree of cure of resins and composites containing benzoyl PFT leads to a higher Knoop hardness, improved transverse strength and better resistence to solvents with varying solubility parameters. Compositions using PFT demonstrate a superior depth of cure which improves a composite resin filling in a property that is desirable.

These properties are illustrated in the following tables:

Table I illustrates the depth of cure (DOC) obtained by various molar amounts of PFT in a composite comprising the resin described above (NCO/TEGMA) 25% by weight, filled with 75% by weight barium glass, as compared with the same composite using camphorquinone (CQ) as an initiator. A sample of each composite was tested after irradiation after 10 seconds and after 40 seconds using a PRISMITICS® light.

The table illustrates that much smaller molar amounts of PFT are effective as an initiator as compared to camphorquinone.

The depth of cure (DOC) was determined by irradiating composite material in rectangular molds (2.5 x 3.5 x 10 mm) for 40 seconds with a PRISMETICS® Light.

To approximate the depth of cure, the specimen is removed from a mold. The soft, uncured composite material is removed from the underside of the cylinder and the hardness of the bottom surface measured. Uncured, soft material is further removed from the underside until a hardness value of 70 is obtained, which approximates acceptable curing.

TABLE I

| Wt. % in Composite PFT | M Mole | Depth of Cure (mm) | |
|---|---|---|---|
| | | 10 Sec. | 40 Sec. |
| 0.025 | 0.04849 | 0 | 3.5 |
| 0.05 | 0.09765 | 3.8 | 5.9 |
| 0.1 | 0.1953 | 4.3 | 5.9 |
| 0.15 | 0.29295 | 4.0 | 5.0 |
| CQ | | | |
| 0.0375 | 0.2259 | 0 | 1.5 |
| (measurements made 24 hours after light activation) | | | |

Table II illustrates the percent conversion obtained using various amounts PFT as compared to CQ. The table illustrates that it requires about a five fold molar amount of CQ to obtain an equivalent % conversion obtained by PFT. The table also illustrates that an enhanced % of conversion is obtained when PFT is used together with BPO, with Lucerin (LR) and with EDAB.

The degree of conversion was determined on thin resin films using FTIR spectroscopy. The films were prepared between Mylar sheets, irradiated for 10 seconds with the Prismetics Light and stored ina dry oven for 24 hours between the irradiation and the IR-measurements to allow the completion of post curing.

In the calculation of the proportion of double bonds converted, the urethane absorption at about 3350 wavenumbers was used as an internal standard to calibrate the reduction of the vinyl absorption at 1637 wavenumbers.

Ethyl 4 (N,N-dimethylaminobenzoate)

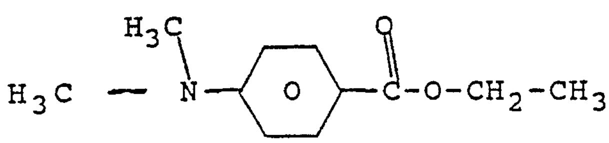

(EDAB)

is used as an accelerator. Percentages are percentages by weight. Test data illustrate improved conversion of monomer, when an amine accelerator is used.

TABLE II

| Wt. % in Composite | m moles | | % Conversion |
|---|---|---|---|
| CQ alone | | | |
| 0.0375 | .2259 | | Not Measureable |
| 0.075 | .4518 | | 53 |
| PFT | | | |
| 0.05 | .09765 | | 53 |
| 0.05 | | + BPO glass (1% on glass, 0.75% in composite) | 65 |
| 0.05 | | +1% LR (0.25% in composite) | 75 |
| 0.05 | | +0.8% EDAB (0.20% in composite) | 66 |

The data illustrated in the table is suggestive of the improved results than may be obtained when PFT is used together in a composite with Lucerin as is illustrated in copending case No. 1590, incorporated herein by reference.

Table III illustrates the Knoop hardness (KHN) for 3 different composite compositions illustrated in the table. The table illustrates that the addition of PFT to conventional camphorquinone/-amine system provides higher Knoop hardness throughout a greater depth of the sample.

The Knoop hardness profiles were obtained by measuring the Knoop Hardness with a Kentron Micro Hardness Tester every 0.5 mm along the surface of specimens which were cured in rectangular molds as described above. Knoop hardness is determined at various depths similar to the method of determining depths of cure.

Cures were obtained using both PRISMA® and PRISMETICS® Lights, both products of L.D. Caulk, a division of Dentsply International Inc.

TABLE III

| | | Wt. % in Composite | Wt. % in Composite |
|---|---|---|---|
| NCO/TFGDMA (50/50) | | 25 | 25 |
| Filler (glass) | | 75 | 75 |
| PFT (as part of resin) | | 0.05 | 0.0 |
| CQ (as part of resin) | | 0.0375 | 0.0375 |
| EDAB (as part of resin) | | 0.200 | 0.200 |
| Profile: (each 0.5mm) | | KHN(kg/cm$^2$ | KHN(kg/cm$^2$ |
| | 0.5mm | 55 | 45 |
| | 1.0 | 55 | 43 |
| | 1.5 | 55 | 36 |
| | 2.0 | 53 | 33 |
| | 2.5 | 44 | 27 |
| | 3.0 | 36 | 20 |
| | 3.5 | 24 | 14 |
| | 4.0 | 10 | -- |
| | 4.5 | -- | -- |
| | 5.0 | -- | -- |

It has also been found that trace amounts of metal ions may also be helpful in the curing efficiency of this system. For example, copper in the +2 state or other redox metals ions may be useful.

Table IV illustrates the increased Depth of Cure that is obtained in compositions containing an acid as compared to similar compositions which use PFT, PFT and LR, and each of PFT and LR in combination with peroxides, amines and butylated hydroxy toluene (BHT). The Knoop hardness of each composite tested were measured at depths 0.5 mm - 5.0 mm from the surface of the composite and the measurements obtained are illustrated in the rows at right of the table. The weight percentage of resin, filler, and other components of the composite are illustrated in the column at the left of the table.

Milled Raysorb is barium boron aluminum silicate glass milled to 3-5 microns.

BME in the table is Benzoin methyl ether.

TABLE IV

| | Composite % | DoC mm | KNOOP Profile .5 | 1.0 | 2.0 | 3.0 | 4.0 |
|---|---|---|---|---|---|---|---|
| Ex. 1 | SV3-176-1C | | not measurable | | | | |
| NCO/TEGOMA | 24.93 | | | | | | |
| PFT | .07 | | | | | | |
| Milled Raysorb | 75.00 | | | | | | |
| BPO | .00 | | | | | | |
| Ex. 2 | SV3-177-1C | | not measurable | | | | |
| NCO/TEGOMA | 24.92 | | | | | | |
| PFT | .07 | | | | | | |
| BHT | .002 | | | | | | |
| Milled Raysorb | 75.00 | | | | | | |
| BPO | .00 | | | | | | |
| Ex. 3 | SV3-177-2C | | 20.2 | 18.4 | 19.0 | 17.0 | 23.6 |
| NCO/TEGOMA | 24.92 | | | | | | |
| PFT | .07 | | | | | | |
| BHT | .002 | | | | | | |
| Milled Raysorb | 74.26 | | | | | | |
| BPO | .74 | | | | | | |
| Ex. 4 | SV3-177-3C | | - | - | - | 3.7 | 3.2 |
| NCO/TEGOMA | 24.32 | | | | | | |
| PFT | .07 | | | | | | |
| penta | .61 | | | | | | |
| Milled Raysorb | 75.00 | | | | | | |
| BPO | .00 | | | | | | |
| Ex. 5 | SV3-179-4C | | - | - | 4.4 | 15.0 | 14.0 |
| NCO/TEGOMA | 24.68 | | | | | | |
| PFT | .07 | | | | | | |
| PTSA | .25 | | | | | | |
| Milled Raysorb | 75.00 | | | | | | |
| BPO | .00 | | | | | | |
| Ex. 6 | SV3-173-1C | | 49.6 | 40.3 | 10.3 | - | - |
| NCO/TEGOMA | 24.68 | | | | | | |
| PFT | .07 | | | | | | |
| BHT | .00 | | | | | | |
| LR | .25 | | | | | | |
| Milled Raysorb | 75.00 | | | | | | |
| BPO | .00 | | | | | | |

TABLE IV continued

| | | | | | | |
|---|---|---|---|---|---|---|
| Ex. 7 | SV3-182-3C | 51.6 | 33.8 | 11.4 | 5.0 | - |
| NCO/TEGOMA | 24.68 | | | | | |
| PFT | .07 | | | | | |
| BHT | .002 | | | | | |
| LR | .25 | | | | | |
| Milled Raysorb | 75.00 | | | | | |
| BPO | .00 | | | | | |
| Ex. 8 | SV3-182-2C | 49.4 | 38.6 | 19.6 | 11.1 | - |
| NCO/TEGOMA | 24.63 | | | | | |
| PFT | .07 | | | | | |
| BHT | .002 | | | | | |
| LR | .25 | | | | | |
| CHP | .05 | | | | | |
| Milled Raysorb | 75.00 | | | | | |
| BPO | .00 | | | | | |
| Ex. 9 | SV3-182-1C | 15.2 | 13.3 | 11.1 | 10.9 | - |
| NCO/TEGOMA | 24.87 | | | | | |
| PFT | .07 | | | | | |
| BHT | .002 | | | | | |
| LR | .00 | | | | | |
| CHP | .05 | | | | | |
| Milled Raysorb | 75.00 | | | | | |
| BPO | .00 | | | | | |
| Ex. 10 | SV3-182-4C | 47 | 29 | 6.70 | 5.20 | 5.40 |
| NCO/TEGOMA | 24.08 | | | | | |
| PFT | .07 | | | | | |
| BHT | .002 | | | | | |
| LR | .24 | | | | | |
| penta | .60 | | | | | |
| Milled Raysorb | 75.00 | | | | | |
| BPO | .00 | | | | | |
| Ex. 11 | SV3-183-1C | 45.2 | 41.4 | 18.4 | 17.9 | 17.1 |
| NCO/TEGOMA | 24.04 | | | | | |
| PFT | .07 | | | | | |
| BHT | .002 | | | | | |
| LR | .24 | | | | | |
| CHP | .05 | | | | | |
| penta | .60 | | | | | |
| Milled Raysorb | 75.00 | | | | | |
| BPO | .00 | | | | | |

TABLE IV continued

| | | | | | | |
|---|---|---|---|---|---|---|
| Ex. 12 | SV3-173-16C | 45.2 | 22.3 | 15.0 | 9.7 | - |
| NCO/TEGOMA | 24.44 | | | | | |
| PFT | .07 | | | | | |
| LR | .24 | | | | | |
| BFA | .24 | | | | | |
| Milled Raysorb | 75.00 | | | | | |
| BPO | .00 | | | | | |
| Ex. 13 | SV3-177-5C | 32.0 | 35.0 | 41.0 | 42.0 | 43.0 |
| NCO/TEGOMA | 24.63 | | | | | |
| PFT | .07 | | | | | |
| LR | .00 | | | | | |
| BFA | .25 | | | | | |
| CHP | .05 | | | | | |
| Milled Raysorb | 75.00 | | | | | |
| BPO | .00 | | | | | |
| Ex. 14 | SV3-178-1C | 39.6 | 24.0 | 11.0 | 5.0 | - |
| NCO/TEGOMA | 24.46 | | | | | |
| PFT | .07 | | | | | |
| BME | .22 | | | | | |
| LR | .24 | | | | | |
| Milled Raysorb | 75.00 | | | | | |
| BPO | .00 | | | | | |
| Ex. 15 | SV3-173-5C | 22.0 | 16.0 | 11.0 | 8.5 | - |
| NCO/TEGOMA | 24.49 | | | | | |
| PFT | .07 | | | | | |
| BME | .24 | | | | | |
| 4-EDAB | .20 | | | | | |
| Milled Raysorb | 75.00 | | | | | |
| BPO | .00 | | | | | |
| Ex. 16 | SV3-173-3C | 43.8 | 43.8 | 18.4 | - | - |
| NCO/TEGOMA | 24.49 | | | | | |
| PFT | .07 | | | | | |
| LR | .24 | | | | | |
| 4-EDAB | .20 | | | | | |
| Milled Raysorb | 75.00 | | | | | |
| BPO | .00 | | | | | |

BFA 3,5-bis-(trifluoromethyl) benzoic acid
PTSA p-toluenesulfonic acid
penta dipentaerythritol pentaacrylate phosphoric acid ester
CHP cumene hydroperoxide
BHT butylated hydroxytoluene
\- KHN is too low to be measured precisely

Using PFT alone as an initiator, the Knoop hardness of the composite was not measurable. (See examples 1 and 2). By comparison, the use of penta and PTSA in an otherwise similar composition increases the cure at depths of 2, 3 and 4 mm. (See examples 4 and 5). Similar results are seen when examples 10 and 11 (containing acid) are compared with similar compositions in examples 7 and 8 (compositions without acid); and examples 12 and 13 (containing acid) compared with examples 7 and 8 (in which the acid is replaced by BHT).

While present embodiments of the invention and methods practicing the same have been illustrated and described, it will be recognized by those skilled in the art that this invention may be otherwise variously embodied and practiced within the scope of the following claims.

## Claims

1. A photopolymerizable composition for dental use comprising at least one olefinically unsaturated monomer and a catalyst comprising a titanate of formula I

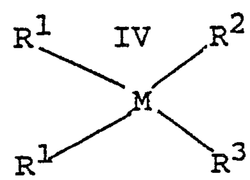

wherein M is a tetravalent titanium atom, $R^1$ is cyclopentadienyl, idenyl or said cyclopentadienyl substituted by alkyl of 1 to 6 carbon atoms, $R^2$ and $R^3$ are each the same and are each phenyl which is substituted by fluorine atoms in at least one of the two ortho-positions relative to the metal-carbon bond, or $R^2$ and $R^3$ together are a radical of the formula III

-Q-Y-Q-

wherein Q is a phenylene ring and each of the two bonds is in the ortho-position to the Y group and each meta-position to the Y group is substituted by a fluorine atom; or wherein said phenyl ring of $R^2$ and $R^3$ and phenylene ring of Q is further substituted by halogen; by amino which is unsubstituted or substituted by alkyl of 1 to 2 carbon atoms or quaternized with an alkyl halide of up to 12 carbon atoms; by pyrrolidono, by piperidino, by piperazino, by morpholino or by N-methylpiperazino; or by aminoalkyl of up to 6 carbon atoms wherein the amino group is unsubstituted or substituted by alkyl of up to 12 carbon atoms or quaternized with an alkyl halide of up to 12 carbon atoms, and Y is methylene, ethylidene, propylidene, a direct bond, O or S, with the proviso that, when $R^2$ and $R^3$ are each pentafluorophenyl, $R^1$ is cyclopentadienyl substituted by alkyl of 1 to 6 carbon atoms.

2. A composition according to claim 1 wherein $R^2$ and $R^3$ each are unsubstituted or substituted 2,6-difluorophenyl, or $R^2$ and $R^3$ to together are an unsubstituted or a substituted radical of the formula

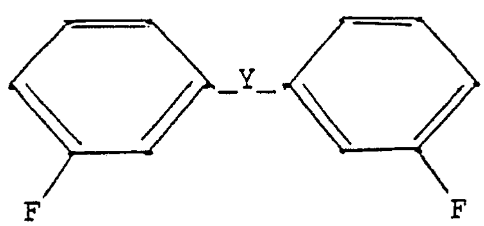

3. A composition according to claim 1, wherein $R^2$ and $R^3$ have the same meaning.

4. A composition according to claim 3, wherein $R^2$ and $R^3$ are 2,6-difluorophenyl which contains 1 to 3 further substituents.

5. A composition according to claim 1, wherein $R^1$ is cyclopentadienyl or $C_1$-$C_4$-alkyl-substituted cyclopentadienyl, and $R^2$ and $R^3$ are a radical of the formula

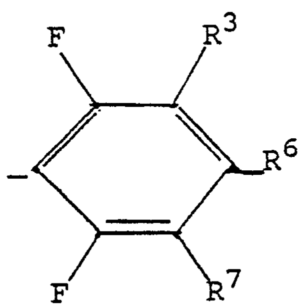

wherein each of $R^5$, $R^6$ and $R^7$ independently is a hydrogen atom, R, Cl, Br, a tertiary amino group, a tertiary aminoalkyl group, a quaternary ammonium group or quaternary ammoniumalkyl group, with the proviso that only two of $R^5$, $R^6$ and $R^7$ are fluorine.

6. A composition according to claim 5, wherein $R^6$ is fluorine, $R^5$ is H, fluorine, chlorine or bromine, and $R^7$ is H, Cl or Br, or $R^5$ and $R^7$ are H, fluorine, chlorine or bromine and $R^6$ is H chlorine, bromine, a tertiary amino group, a tertiary aminoalkyl group or a quaternary ammonium or ammoniumalkyl group.

7. A composition according to claim 1 wherein said titanate is Bis-pentafluorophenyl-Bis-cyclopentadienyl titanate.

8. The composition of claim 1 which includes perioxide.

9. The composition of claim 1 wherein the monomer includes at least one acrylic or methacrylic ester.

10. The composition of claim 1 which includes a tertiary amine.

11. The composition of claim 1 wherein the composition contains a finely divided inorganic filler.

12. The use of the composition of claim 1 for treating teeth by its application to a prepared tooth.

13. The composition of claim 1 which comprises a catalyst system including vicinal diketone and amine.

14. The composition according to claim 13 in which said vicinal diketone is camphorquinone.

15. The composition of claim 1 which comprises acylphenyl phosphonate.

16. A method of curing resin materials for dental application comprising

(a) including an effective amount of at least one olefinically unsaturated monomer and a catalyst comprising a titanate of formula I

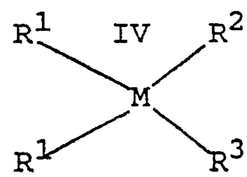

wherein M is a tetravalent titanium atom, $R^1$ is cyclopentadienyl, idenyl or said cyclopentadienyl substituted by alkyl of 1 to 6 carbon atoms, $R^2$ and $R^3$ are each the same and are each phenyl which is substituted by fluorine atoms in at least one of the two ortho-positions relative to the metal-carbon bond, or $R^2$ and $R^3$ together are a radical of the formula III

-Q-Y-Q

wherein Q is a phenylene ring and each of the two bonds is in the ortho-position to the Y group and each meta-position to the Y group is substituted by a fluorine atom; or wherein said phenyl ring of $R^2$ and $R^3$ and phenylene ring of Q is further substituted by halogen; by amino which is unsubstituted or substituted by alkyl of 1 to 2 carbon atoms or quaternized with an alkyl halide of up to 12 carbon atoms; by pyrrolidino, by piperidino, by piperazino, by morpholino or by N-methylpiperazino; or by aminoalkyl of up to 6 carbon atoms wherein the amino group is unsubstituted or substituted by alkyl of up to 12 carbon atoms or quaternized with an alkyl halide of up to 12 carbon atoms, and Y is methylene, ethylidene, propylidene, a direct bond, O or S, with the proviso that, when $R^2$ and $R^3$ are each pentafluorophenyl, $R^1$ is cyclopentadienyl substituted by alkyl of 1 to 6 carbon atoms in said resin material

(b) applying actinic light in the visible wavelength range to said resin material.

17. The method of claim 16 comprising the step of using Bis-pentafluorophenyl Bis-cyclopentadienyl titanate as an initiator.

18. The method of claim 17 comprising the step of using peroxide as a coinitiator.

19. The method of claim 17 comprising the step of using acylphenyl phosponate as a coinitiator.

20. The method of claim 17 comprising the step of using vicinal diketone as a coinitiator.